# EUROPEAN PATENT APPLICATION

(11) **EP 1 707 194 A1**
(43) Date of publication of application: **04.10.2006**
(21) Application number: 05709255.3
(22) Date of filing: 21.01.2005
(51) Int. Cl.: A61K 31/198, A61P 25/22, A23L 1/305

(54) **STRESS RELIEVING COMPOSITION**

(30) Priority: 23.01.2004 JP 2004016249
(71) Applicant: Ajinomoto Co., Inc., Tokyo 104-8315 (JP)
(72) Inventor: SMRIGA, Miroslav, Kawasaki-shi, Kanagawa 210868 1 (JP); ANDO, Toshihiko, Ajinomoto Co., Inc., Tokyo 1048315 (JP); MORINAGA, Yasushi, Ajinomoto Co., Inc., Tokyo 1048315 (JP)
(74) Representative: Nicholls, Kathryn Margaret
(86) International application number: PCT/JP2005/000748
(87) International publication number: WO 2005/070408

(57) **Abstract**

A composition for relieving stress and/or disorder caused by stress is provided, which composition comprises lysine and arginine as active ingredients. The lysine and arginine may be in the form of a salt. In a preferred mode, the cause of stress is any one selected from the group consisting of mental strain, repetitive work, intellectual labor, menopausal mental instability, anxiety and strain with respect to a future event and premenstrual mental instability and strain.

## Description

### Technical Field

The present invention relates to a composition for relieving stress and/or disorder caused by stress and a method of relieving stress for a person who uses such a composition. The composition of the present invention contains lysine and arginine as active ingredients, and can be used as a food and drink, or a health supplement.

### Background Art

As for several types of food components, a test for examining the presence or absence of an action of suppressing mental stress is done using normal individuals as subjects. As a result, it has been reported that what are the best components among them were two types of amino acids, tryptophan and tyrosine. Because tryptophan, which is an essential amino acid, is the precursor of serotonin, a concept that tryptophan (Trp) is used for the purpose of relieving mental stress can be intuitively understood. When Trp in the brain is supplied to serotonin-producing neurons, the serotonin production rate in such neurons is affected. Trp in the brain is adversely affected by chronic stress (see, for example, Non-patent document 1).

A comparative clinical study shows that when a Trp supplement is taken alone or together with carbohydrates, stress-induced mood is relieved (see, for example, Non-patent document 2). On the other hand, however, there is also a report that as a result of a clinical study on Trp conducted recently, the mood enhancement effect of Trp could not be fully supported (see, for example, Non-patent document 3).

The reason why tyrosine (Tyr) is considered to be a nutritional supplement having an anti-stress action is similar to the case of Trp. Tyr is the precursor of dopamine, epinephrine and norepinephrine, which are catecholamine neurotransmitters. These neurotransmitters control mood against psychosocial and mental stress. In the case of neurons that act actively, when Tyr concentration in the neurons increases, norepinephrine production is stimulated in the neuronal terminal. However, in the case of neurons that do not act, it is not stimulated (see, for example, Non-patent document 4). However, the relationship between Tyr in the blood and Tyr in the brain is complicated, and the use of a Tyr nutritional supplement for the purpose of regulating stress is not well founded. Tyr has already been tested in patients with depression, and a significant effect in terms of mood criterion was not observed (see, for example, Non-patent document 5). However, when a study was carried out using normal subjects exposed to mental stress, Tyr improved the mood significantly (see, for example, Non-patent document 6).

By putting these together, the clinical studies do not conclusively support or refute the effectiveness of Trp and Tyr supplements for stress. Further, there is a need to carefully evaluate nutritional supplements containing Trp or Tyr from the viewpoint of side effects (such as diarrhea and heart rate turbulence).

The animal study performed recently by us shows that when feed deficient in L-lysine (Lys), which is another amino acid, was given to animals, stress-induced anxiety or defecation is increased (see, for example, Non-patent document 7). Further, with the use of rats fed with normal diet, by administration of L-lysine hydrochloride + L-arginine (p.o.) for 5 days, significant improvement of scoring of anxiety symptoms in models with stress-induced anxiety disorder was observed (see, for example, Non-patent document 8).

When a binding activity of L-lysine (0.8 mmol/dl) to 5-HT4 receptor was examined under the conditions free from the effect of 5-HT1A, 2A, 2B, 2C and 3 receptors, L-lysine (0.07 and 0.7 mmol/dl) blocked 5-HT-induced contraction of the guinea pig ileum *in vitro* (P<0.05, P<0.01). L-lysine (1 g/kg, p.o.) significantly suppressed anxiety disorder of rats induced by a 5HT-4 receptor agonist (3.0 mmol/l.s.e.) *in vitro.* L-lysine has a so-called blocking effect, that is, it partially serves as a 5HT-4 receptor antagonist and strongly suppresses an intestinal disease or anxiety disorder mediated by 5HT-4 receptor. Further, L-lysine is also a benzodiazepine receptor agonist (see, for example, Non-patent document 9). Moreover, amino acid metabolic overreaction caused by stress is improved to a normal state by L-lysine. Further, L-lysine improves hyperammonemia caused by body protein degradation. However, when L-lysine hydrochloride is given during intense stress, the required amount of L-arginine is increased (see, for example, Non-patent document 10).

When the anti-stress action of L-lysine hydrochloride and L-arginine was examined using broilers based on the results as described above, the body weight of the broilers fed with diet supplemented with L-lysine hydrochloride and L-arginine decreased less than the broilers of the control exposed to an equivalent stress. It was clearly revealed that the percentage of body fat is also suppressed in the same manner. It is considered that a L-lysine hydrochloride and L-arginine supplement increases the effectiveness of a broiler farm, and the data indicates the possibility that by using Lys and Arg in combination, a stress-induced disease such as anxiety can be prevented in a patient or a test subject who feels chronically sick (see, for example, Patent document 1).

However, it has not been known whether or not the above-mentioned various food components can relieve such stress even for an individual who is healthy except for having daily mild mental stress.

Patent document 1: International Publication No. WO 02/076445
Non-patent document 1: Young et al., Neuropsychopharmacology, 2000, 23, 411-418
Non-patent document 2: Maes et al., Neuropsychopharmacology, 1999, 20, 188-197
Non-patent document 3: Van der Does, Journal of Affective Disorders, 2001, 64, 107-119
Non-patent document 4: Wurtman et al., Pharmacological Revue, 1980, 32, 315-335
Non-patent document 5: Gelenberg et al., Journal of Affective Disorders, 1990, 19, 125-132
Non-patent document 6: Deijin and Orlebeke, Brain Research Bulletin, 1994, 33, 319-323
Non-patent document 7: Journal of Nutrition, 2002, 132, 3744-3746
Non-patent document 8: Nutr. Neurosci., 2003, 7, 125-128
Non-patent document 9: Eur. J. Pharmacol., 1993, 233, 209-217 Non-patent document 10: Smriga, M. and Torii, K. Amino Acids, 2003, 24, 435-437

### Disclosure of the invention

### Problems that the Invention is to Solve

An object of the present invention is to provide a novel composition effective in relieving daily stress. Further, another object is to provide such a composition as a nutritional supplement or a food which is commercially available to the public.

### Means for Solving the Problems

In order to solve the above-mentioned problems, the present inventors have made intensive studies, and as a result, they found that a composition containing lysine and arginine in combination (hereinafter sometimes referred to as the "composition of the present invention") has an action of relieving daily stress, thus the present invention has been completed. The present invention includes the following items.

(1) A composition for relieving stress and/or disorder caused by stress, comprising lysine and arginine as active ingredients. Lysine and arginine can be used in the free form, however, they may be in the form of a salt, or a mixture thereof (including a mixture of a plurality of salts, a mixture of one or more types of salts and free forms and the like) (in this description, the free form and its salt forms are collectively called "lysine" and "arginine"). Further, as for an optical isomer thereof, the L-form which is present *in vivo* is preferred.
(2) The composition according to (1), wherein the cause of the stress is any one selected from the group consisting of mental strain, repetitive work, intellectual labor, menopausal mental instability, anxiety or strain with respect to a future event and premenstrual mental instability or strain.
(3) The composition according to (1), wherein the cause of the stress is mental anxiety with respect to a future event which is any one selected from the group consisting of a presentation in front of people, a test, car driving, repetitive work, intellectual labor, menopause and menstruation.
(4) The composition according to (1), wherein the disorder is any one selected from the group consisting of irritation, social anxiety disorder, mental fatigue and sleep disorder.
(5) The composition according to any one of (1) to (4), wherein the mass ratio of lysine to arginine is 1:0.1 to 1:2.
(6) The composition according to any one of (1) to (5), wherein the daily dose contains 0.5 to 20 g of lysine.
(7) The composition according to any one of (1) to (6), wherein the lysine and arginine are in the L-form.
(8) A food and drink or health supplement, containing the composition according to any one of (1) to (7).
(9) A stress emollient (relieving agent), comprising the agent contains lysine and arginine as active ingredients and being administered to a person who is susceptoble to a stressor at least once before occurrence of the stressor. Lysine and arginine may be in the form of a salt.
(10) The stress emollient (relieving agent) according to (9), wherein the stressor is any one selected from the group consisting of a presentation in front of people, a test, car driving, repetitive work, intellectual labor, menopausal mental instability and premenstrual mental instability or strain.
(11) The stress emollient (relieving agent) according to (9) or (10), wherein the lysine and arginine are in the L-form.
(12) A health supplement, consisting of a composition comprising lysine and arginine at a mass ratio of 1:0.1 to 1:2. Lysine and arginine may be in the form of a salt.
(13) The health supplement according to (12), wherein the lysine and arginine are in the L-form.
(14) Use of lysine and arginine or a salt thereof for the manufacture of a stress emollient for suppressing a stress response reaction caused by a stressor.
(15) The use according to (14), wherein the stressor is any one selected from the group consisting of a presentation in front of people, a test, car driving, repetitive work, intellectual labor, menopausal mental instability and premenstrual mental instability or strain.
(16) The use according to (14) or (15), wherein the lysine and arginine are in the L-form.
(17) Use of lysine and arginine or a salt thereof for the manufacture of a stress emollient for administering lysine and arginine contained at a mass ratio of 1:0.1 to 1:2 in such a manner that 0.5 to 20 g of lysine is taken at least once per day before or during the period when mental stress or mental pressure increases.
(18) The use according to (17), wherein the lysine and arginine are in the L-form.

### Advantage of the Invention

By being administered to or taken by an individual who is sensitive to any of various stressors, the composition of the present invention can exert an effect to relieve anxiety, induce relaxation, relieve daily stress, improve health condition (well-being), improve sleep pattern during the period when mental pressure is applied, normalize hormone response to stress, improve gastric sensation before occurrence of a stress factor, reduce visceral fat, or the like.

### Brief Description of the Drawings

[Fig. 1] Fig. 1 is a schematic diagram showing the mechanism of stress response according to the present invention.
[Fig. 2] Fig. 2 is a diagram showing the schedule and observation items of the stress load test in Example.
[Fig. 3] Fig. 3 is a diagram showing the schedule of the Intellectual stress test on the test day in the stress load test in Example.
[Fig. 4] Fig. 4 is a diagram showing the measurement results of anxiety levels (STAI-X2) in a daily life.
[Fig. 5] Fig. 5 is a diagram showing the measurement results of questionnaires (STAI-X1) of current stress mode.
[Fig. 6] Fig. 6 is a diagram showing the measurement results of saliva cortisol in the stress load test in Example.

### Best Mode for Carrying Out the Invention

Hereinafter, embodiments of the present invention will be described.

### (With regard to stress response related to the present invention)

The mental stress is a collective term of forces of a deleterious nature that tend to disturb normal physiological equilibrium (homeostasis) without physically affecting the body, and is sometimes referred to as distress that causes maladaptation, as opposed to good stress which induces favorable adaptation (Stedman's Concise Medical & Allied Health Dictionary 3rd ed., 1997). On the other hand, the stressor means an event that triggers stress response, a stress factor or a stress source.

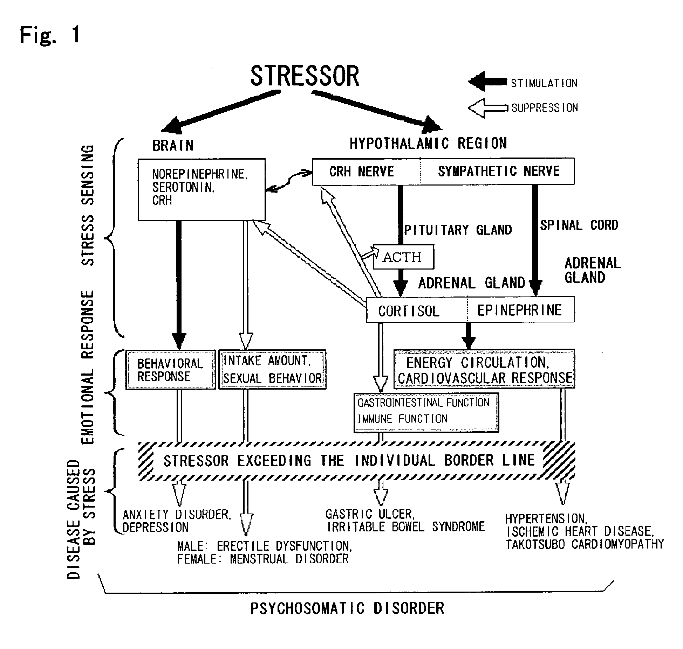

Fig. 1 is a diagram for explaining stress, a stressor and stress response. In the present invention, it is considered that a stress system has evolved to increase a chance for higher organisms to survive when they encounter an event (such as encounter a predator) that threatens their survival. A certain level of stressor is important even at present, but a chronic mental stressor stimulation exceeding a certain level causes a pathological physiological response. The border line of the two, i.e., whether it is a stressor within the acceptable limit or it is a pathological stressor varies depending on the individual, for example, nutritional conditions, genetic factors, social life style or the like.

There are numerous stressors either mentally or physically. It is because the life style has changed since around the 1960's and the number and quality of mental stimulations have dramatically increased. It has been reported that 19.1 millions of American adults go to hospital or do not feel good because of stress (Narroe et al., NIMH epidemiology note: prevalence of anxiety disorders, 1998). Basically, stress response is nonspecific, that is, there is a regular pattern in stress response as shown in Fig. 1 regardless of the type of stressor (Depression and Anxiety, 2000, 12, 2-19). The white arrows indicate suppression, and the black arrows indicate stimulation. A stressor activates corticotropin-releasing hormone (CRH) in the hypothalamic region and a hypothalamic sympathetic nerve. These systems induce an adrenal cortical hormone (cortisol or epinephrine) thereby activating the function of cardiovascular systems, making energy circulation smooth, increasing a chance of running away, and confronting the stressor. In addition to this, the norepinephrine system, serotonin system or CRH nerve in higher order brain is activated, and responsive action suitable for the stressor is brought about. On the other hand, during the presence of the stressor, responses that are not important for the survival (food intake, immunity or sexual behavior) are suppressed (Neuropsychopharmacology, 2002, 26, 358-367).

However, if the stressor is intense or persistent, "normal stress response" changes to a pathological stress response (Fig. 1). The term "stress susceptibility" or "stress sensitivity" in the present invention refers to a degree of stress response reaction to any of various stressors as described above, and the sensitivity (the degree of stress response reaction) varies depending on the specific stressor and the individual who receives the stressor in some cases. Therefore, a preferred embodiment of the present invention is characterized in that by administering the composition of the present invention to an individual who is sensitive to a specific stressor, a stress response reaction caused by the stressor is suppressed. As described above, there are a number of stressors, and examples thereof include presentation, a test, driving, a stressful sport event, business meeting and the like.

### (Composition of the present invention)

Lysine and arginine to be used in the present invention may be any of those obtained by hydrolyzing a naturally occurring protein derived from an animal or a plant, and those obtained by the fermentation method or the chemical synthesis method. In lysine and arginine, as optical isomers, D-form and L-form are present, however, the L-form which is a component of a biological protein is preferably used in the present invention. Lysine and arginine may also be used in the form of any of various salts. As for the salts of lysine and arginine, because these amino acids are basic, salts with an acid are mainly used. As the acid, either an inorganic acid or an organic acid may be used. Examples of the inorganic acid include hydrochloric acid, sulfuric acid, nitric acid, phosphoric acid, hydrobromic acid, and hydroiodic acid. Examples of the organic acid include formic acid, acetic acid, propionic acid, oxalic acid, succinic acid, maleic acid, fumaric acid, citric acid, glutamic acid, asparatic acid, γ-lionlenic acid, tocopherol succinate monoester, tocopherol phosphate, ascorbic acid, ascorbyl phosphate, tocopherol ascorbyl phosphate, thioctic acid, N-acetylcysteine, N,N'-diacetylcysteine, and lipoic acid .

The form of lysine and arginine to be used in the present invention may be any as long as they are immediately converted into lysine or arginine in vivo when they are taken. However, examples thereof include peptides. It is preferred that the content of lysine and arginine in the peptides is in the range from 10 to 30% or higher. As the peptide components, lysine and arginine are active components, therefore, they are essential, but the types of amino acids other than these do not matter. The peptide can be obtained by any of various methods such as chemical synthesis, fermentation, hydrolysis of naturally occurring protein, naturally occurring peptides, etc., and any of these can be used.

The mixing ratio of lysine to arginine in the composition of the present invention is in the range from 1:0.1 to 1:2, preferably from 1:0.5 to 1:2, more preferably it is about 1:1 in terms of mass. It is because the lysine concentration in the blood is not affected by stress loading, but the arginine concentration in the blood decreases, therefore by administering lysine alone, the decrease in the arginine concentration in the blood due to stress is amplified. Accordingly, by administering lysine and arginine in combination in the composition of the present invention, the arginine concentration in the blood in the case of stress loading can be maintained, and at the same time, an anti-stress effect of lysine can be exerted. Incidentally, in a preferred embodiment, the composition of the present invention can contain L-lysine in the form of a hydrochloride salt and L-arginine in the free form.

### (Dosage regimen of the composition of the present invention and dosage form of preparation)

The composition of the present invention is taken before or during the period when mental stress or mental pressure increases at least once, and preferably several times at regular time intervals. More preferably, it is taken at least once before a stressor occurs. Examples of such a stressor include presentation, an examination, driving, a stressful sport event, business meeting and the like. There is also a method in which it is taken immediately before mentally harsh conditions.

The dose in the case where the composition of the present invention is administered to an individual who is sensitive to a stressor varies depending on the age of the individual to be administered, the degree of the sensitivity to stress or the like, and cannot be defined comprehensively. However, to a general adult, in the case of oral administration, the daily dose contains the equivalent of 0.1 to 50 g, or more preferably 0.5 to 20 g of lysine in a free form.

The composition of the present invention can be administered or taken in the form of a food and drink, or a health supplement. For example, it can be safely administered orally as a tablet (including a sugar-coated tablet and a film-coated tablet), a powder, a granule, a capsule (including a soft capsule), a syrup, a liquid or the like. In order to produce a preparation for oral administration, in accordance with a known method, lysine and arginine, which are active ingredients, can be formulated into a preparation for oral administration by adding, for example, an excipient (such as lactose, sucrose or starch), a disintegrating agent (such as starch or calcium carbonate), a binder (such as starch, gum arabic, carboxymethyl cellulose, polyvinylpyrrolidone or hydroxypropyl cellulose), or a lubricant (such as talc, magnesium stearate or polyethylene glycol 6000) or the like, and compression molding the mixture, and then as needed, masking the taste, or coating by a known method for the purpose of enteric or sustained performance. As the coating agent, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by Rohm Company, Germany, methacrylic acid-acrylic acid copolymer), a pigment (such as colcothar or titanium oxide) or the like is used. The preparation for oral administration may be either a quick-release preparation or a sustained-release preparation. Alternatively, it can be taken in the form of a common food and drink, and examples thereof include a soft drink, jelly, sweetstuff, juice and the like. However, it is not limited to these.

### Example

Hereinafter, the present invention will be illustrated more specifically with reference to the Example, but the present invention is not limited to this.

A stress load test was carried out in accordance with a test plan as described below.
1. Test design: Double blind between-group comparison study by random allocation
2. Test implementation site: Kawasaki region
3. Number of test subjects: 108 subjects, 54 subjects in each group x 2 groups
4. Form of food: Capsule
   Test diet: L-lysine hydrochloride and L-arginine were used (weight ratio of 1:1).
   Control diet: Tapioca starch was used.
5. Intake amount: In each group, as for each food (No. 0 hard capsules (white)), 6 capsules per administration twice per day, and the total of 12 capsules were taken.
   Test diet (group 1) : L-lysine hydrochloride 2.64 g/day and L-arginine 2.64 g/day were taken.
   Control diet (group 2) : Tapioca starch was taken at 6.00 g/day.
6. Method: In each group, each food was taken twice per day (after breakfast and after supper).
7. Intake period: 7 days
8. Stress test: Intellectual test
   To each of the test subjects, an intellectual stress test (17 minutes) for testing imagination, writing composition skill, calculation speed and analysis skill was given in a noisy place, and a stress marker in saliva was measured at 20 minutes before the test, immediately after the test and at 40 minutes after the test.
9. Evaluation items
   Saliva biochemistry (cortisol)
   Subjective symptoms (anxiety symptoms, stress symptoms)
   Body weight and body fat percentage

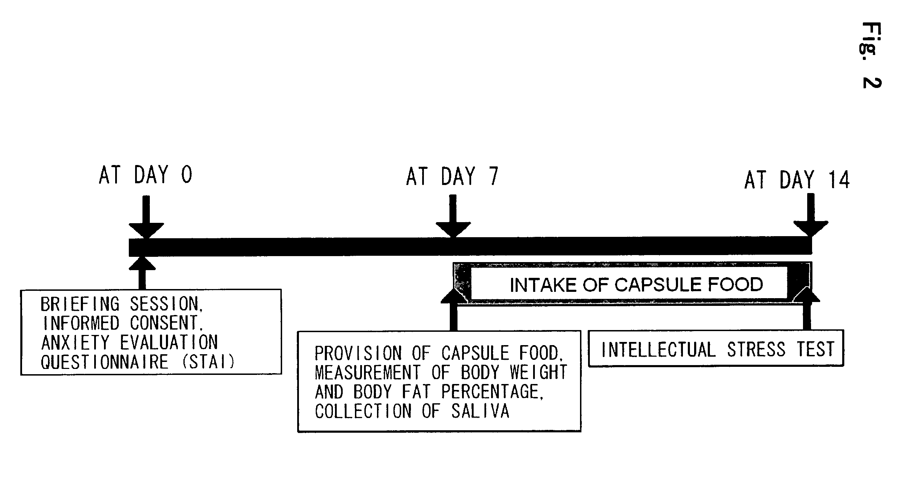

The schedule and the observation items in the stress load test (intellectual stress test) are shown in Fig. 2.

### [At 2 weeks before the initiation of test]

(1) The test subjects were informed of the outline of the test by a doctor and the informed consent was obtained, then, the letter of consent was prepared.
(2) Anxiety evaluation questionnaire (STAI-X1, STAI-X2)
   The test subjects were stratified based on the score of STAI-X1 test performed after the briefing session, and randomly allocated to the placebo group and to the L-lysine hydrochloride + L-arginine group.

### [At 7 days before the test]

(1) The capsule food was handed over.
(2) The body weight and body fat percentage were measured.
(3) Measurement of cortisol in the collected saliva was analyzed at Yanaihara Institute Inc. (Fujinomiya-shi, Shizuoka-ken).

### [From 7 days before the test to the test day]

The capsule food was taken and the self-observation of the body was recorded daily.

### [On the test day]

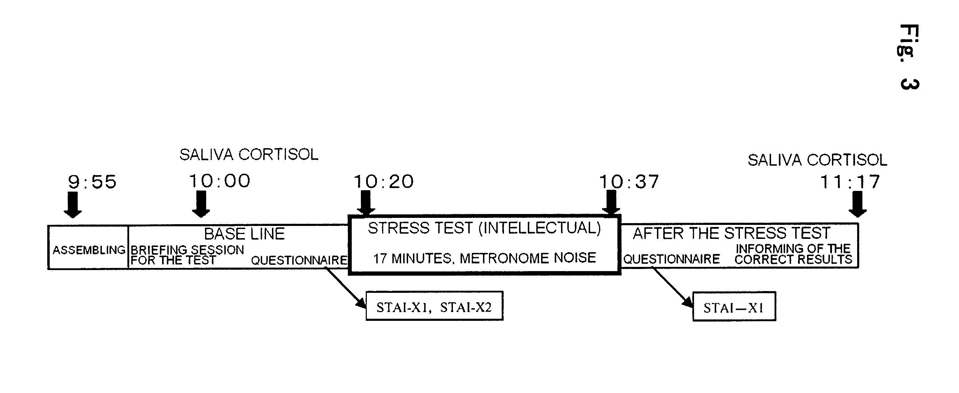

(1) Collection of saliva
   Measurement of cortisol
(2) Questionnaire
   Anxiety evaluation questionnaire (STAI-X1, STAI-X2)
   In order to avoid circadian variation, the stress load test was carried out between 10 o'clock in the morning and noon.
   The times of collecting saliva and performing questionnaire are as shown in Fig. 3.
(3) Measurement of the body weight and the body fat percentage (time: after the last saliva sampling)

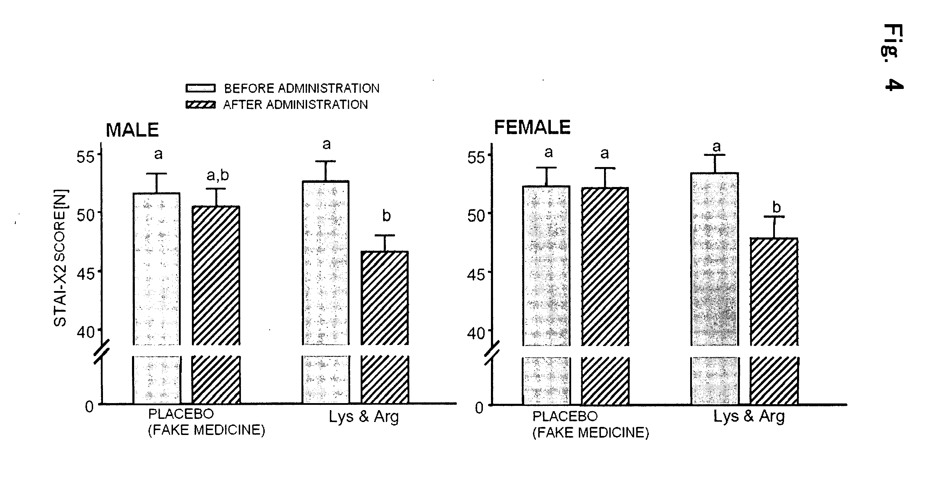

[Results]
The anxiety level in daily life (STAI-X2, with the sexes separated) was measured before and after the intake of capsules. A high score of STAI-X2 indicates a high anxiety level. As shown in Fig. 4, it was revealed that the STAI-X2 score significantly decreased in the group of the test diet (Lys & Arg) (2-way ANOVA, p < 0.05).

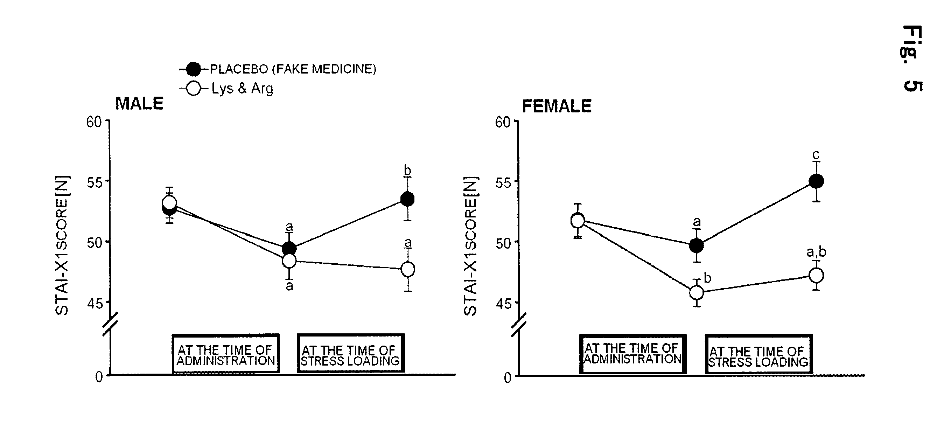

The results of the questionnaires (STAI-X1) showing stress mode at the time of performing questionnaire are shown in Fig. 5 (with the sexes separated). It was revealed that although the STAI-X1 score after the intellectual stress increased, however, the increase was significantly blocked in the group of the test diet (Lys & Arg) (2-way ANOVA, p < 0.05).

Then, the individual intellectual test score was measured (Min = 0%, Max = 100%), and a change due to the intake of capsules was not observed. The score for the placebo group was 55.45 +/- 2.4%; the score for the group of test diet (Lys & Arg) was 53.40 +/- 2.2%.

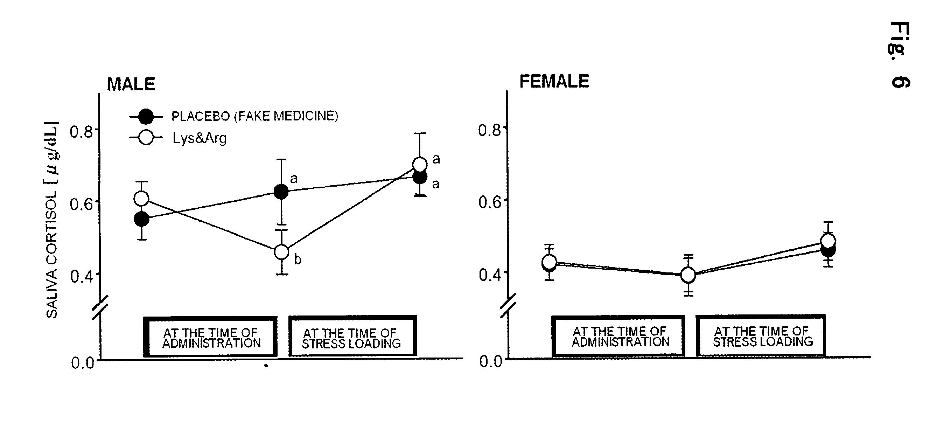

The level of the saliva cortisol is shown in Fig. 6. Although the level of the saliva cortisol before the stress loading in the group of test diet (Lys & Arg) was lower than that in the placebo group, a change in cortisol due to the stress loading was observed only in the group of test diet (Lys & Arg) (2-way ANOVA, p < 0.05). The cortisol in female was lower by 30% than the cortisol in male, and a change due to capsules or stress was also small. In the above test, the body fat percentage in the placebo group was 22.04 +/- 0.98%, and the body fat percentage after the intake of placebo was 22.70 +/-0.99%; the body fat percentage before the intake of the test diet (Lys & Arg) was 21.00 +/- 0.99%, and the body fat percentage after the intake of the test diet (Lys & Arg) was 20.70 +/-1.03%. Further, in the above-mentioned Figs. 4 to 6, as for the letters a, b and c shown in the graphs, different letters indicate a significant difference at a significance level: p < 0.05 (2-way ANOVA).

[Conclusion]

### Anxiety level in daily life

The score of STAI-X2 questionnaire and the baseline saliva cortisol with which the anxiety level in daily life was evaluated were significantly decreased by the test diet (Lys & Arg).

### Mental stress response

It was confirmed that by the intake of the test diet (Lys & Arg) for 7 days, acute mental stress feeling in response to acute mental stress was significantly alleviated compared with the placebo group.

### Industrial Applicability

It was confirmed that the intake of lysine (2.64 g/day) and arginine (2.64 g/day) for 7 days has no side effects and they are a safe combination of amino acids. By administering the composition of the present invention containing lysine and arginine, there is the effect of alleviation of acute stress and anxiety in daily life, and it is useful as a composition for relieving stress.

## Claims

1. A composition for relieving stress and/or disorder caused by stress, comprising lysine and arginine as active ingredients, wherein the lysine and arginine may be in the form of a salt.

2. The composition of claim 1, wherein the cause of said stress is any one selected from the group consisting of mental strain, repetitive work, intellectual labor, menopausal mental instability, anxiety and strain with respect to a future event, and premenstrual mental instability and strain.

3. The composition of claim 1, wherein the cause of said stress is an anxiety with respect to a future event selected from the group consisting of a presentation in front of people, an examination, car driving, repetitive work, intellectual labor, menopause, and menstruation.

4. The composition of claim 1, wherein said disorder is any one selected from the group consisting of irritation, social anxiety disorder, mental fatigue, and sleep disorder.

5. The composition of any one of claims 1 to 4, wherein the mass ratio of lysine to arginine is from 1:0.1 to 1:2.

6. The composition of any one of claims 1 to 5, wherein the lysine is comprised in a daily dose of 0.5 to 20 g on the bases of free form thereof.

7. The composition of any one of claims 1 to 6, wherein the lysine and arginine are in the L-form.

8. Food and drink, or health supplement comprising any one of compositions of claims 1 to 7.

9. A stress emollient comprising lysine and arginine as effective ingredients, which may be in the form of salts,
said stress emollient being administered to a person susceptible to a stressor at least once before occurrence of such stressor.

10. The stress emollient of claim 9, wherein said stressor is any one selected from the group consisting of a presentation in front of people, an examination, car driving, repetitive work, intellectual labor, memopausal mental instability, and premenstrual mental instability and stress.

11. The stress emollient of claim 9 or 10, wherein the lysine and arginine are in the L-form.

12. A health supplement consisting of a composition comprising lysine and arginine at a mass ratio of 1: 0.1 to 1: 2, said lysine and arginine may be in the form of salts.

13. The supplement of claim 12, wherein the lysine and arginine are in the L-form.

14. Use of lysine and arginine or a salt thereof for the manufacture of a stress emollient for suppressing a stress response reaction caused by a stressor.

15. The use of claim 14, wherein said stressor is any one selected from the group consisting of a presentation in front of people, an examination, car driving, repetitive work, intellectual labor, memopausal mental instability, and premenstrual mental instability and stress.

16. The use of claim 14 or 15, wherein the lysine and arginine are in the L-form.

17. Use of lysine and arginine or a salt thereof for the manufacture of a stress emollient for administering lysine and arginine contained at a mass ratio of 1:0.1 to 1:2 in such a manner that 0.5 to 20 g of lysine is taken per day at least once before or during the period when mental stress or mental pressure increases.

18. The use of claim 17, wherein the lysine and arginine are in the L-form.
